# FASCICULE DE BREVET EUROPEEN

(11) **EP 1 381 343 B1**
(45) Date de publication et mention de la délivrance du brevet: **15.12.2004**
(21) Numéro de dépôt: 02727671.6
(22) Date de dépôt: 17.04.2002
(51) Int. Cl.: A61J 7/04

(54) **DISPOSITIF DE PULVERISATION NASALE OU ORALE DE PRODUIT FLUIDE OU PULVERULENT**
VORRICHTUNG ZUR NASALEN ODER ORALEN VERPULVERUNG EINES PULVERIGEN ODER FLUIDEN PRODUKTES
DEVICE FOR NASAL OR ORAL SPRAYING OF A FLUID OR POWDERY PRODUCT

(30) Priorité: 24.04.2001 FR 0105510
(43) Date de publication de la demande: 21.01.2004
(73) Titulaire: VALOIS S.A., 27110 Le Neubourg (FR)
(72) Inventeur: BRUNA, Pascal, F-76300 Sotteville les Rouen (FR)
(74) Mandataire: CAPRI
(86) Numéro de dépôt international: PCT/FR2002/001329
(87) Numéro de publication internationale: WO 2002/085282

(56) Documents cités:
- EP-A- 1 016 399
- WO-A-98/19647
- FR-A- 2 440 021

## Description

La présente invention concerne un dispositif de pulvérisation nasale ou orale de produit fluide ou pulvérulent, et plus particulièrement un tel dispositif commandé électroniquement.

De nos jours, en particulier dans certains domaines de la pharmacie, il existe un besoin pour des dispositifs de pulvérisation de produit fluide ou pulvérulent, en particulier de médicaments, qui doivent permettre non seulement de pulvériser de manière précise et reproductible des doses individuelles de produit, mais qui doivent également remplir d'autres fonctions pour assurer la sécurité et la fiabilité du traitement en cours. En particulier, il est souhaitable de pouvoir empêcher un surdosage, c'est à dire une prise trop répétitive et à des intervalles de temps trop rapprochées de plusieurs doses de médicaments. Une surveillance régulière par le médecin traitant est également souhaitable. Ces dispositifs doivent aussi de préférence être transportables et utilisables n'importe où, pour permettre à l'utilisateur de suivre un traitement précis sans nécessairement être bloqué en un lieu, tel qu'un hôpital.

La présente invention a donc pour but de fournir un tel dispositif qui soit simple et peu coûteux à fabriquer et à assembler.

En particulier, la présente invention a pour but de fournir un tel dispositif qui puisse aisément s'adapter aux dispositifs de distribution classiques utilisés actuellement, sans nécessité de modifications structurelles importantes desdits dispositifs.

La présente invention a donc pour objet un dispositif de pulvérisation nasale ou orale de produit fluide ou pulvérulent comportant un corps, au moins un réservoir de produit contenant une ou plusieurs doses de produit à distribuer, des moyens de distribution actionnés manuellement, tels qu'une pompe ou une valve, et un élément d'actionnement manuel adapté à actionner lesdits moyens de distribution pour distribuer des doses de produit, caractérisé en ce que ledit dispositif comporte un système de solidarisation qui est mobile entre une position embrayée, dans lequel ledit élément d'actionnement manuel coopère avec lesdits moyens de distribution, et une position débrayée, dans laquelle ledit élément d'actionnement manuel ne coopère pas avec lesdits moyens de distribution, ledit système de solidarisation étant commandé par une unité de commande électronique paramétrable.

Avantageusement, ledit système de solidarisation comporte un élément mécanique, tel qu'une tige, mobile entre une position embrayée dans laquelle il relie mécaniquement ledit élément d'actionnement avec un organe solidaire des moyens de distribution de telle sorte qu'un actionnement dudit élément d'actionnement entraîne la distribution d'une dose de produit, et une position débrayée dans laquelle il n'y a pas de liaison entre l'élément d'actionnement et les moyens de distribution, de telle sorte qu'un actionnement dudit élément d'actionnement n'entraîne pas de distribution de produit.

Avantageusement, ledit élément d'actionnement est un bouton monté pivotant ou coulissant sur un côté latéral dudit corps, ladite tige étant disposée dans ledit bouton d'actionnement, une extrémité de ladite tige coopérant, dans la position embrayée, avec un orifice de réception dudit organe solidaire des moyens de distribution.

Avantageusement, lesdits moyens de distribution sont réalisés sous la forme d'une pompe ou d'une valve, ledit organe solidaire des moyens de distribution étant relié au piston de la pompe, respectivement à la soupape de la valve.

Avantageusement, ladite unité de commande comporte un microprocesseur et des moyens d'alimentation en énergie.

Avantageusement, ledit dispositif comporte des moyens de signalisation, tels qu'un afficheur LCD ou des avertisseurs sonores, pour informer l'utilisateur de l'état du dispositif.

Avantageusement, ladite unité de commande est paramétrable à distance, notamment au moyen d'un ordinateur, et comporte des moyens de transmission et de réception de données.

Avantageusement, ladite unité de commande est paramétrable pour permettre respectivement empêcher l'actionnement du dispositif selon plusieurs fonctions prédéterminables.

Avantageusement, lesdites fonctions prédéterminables comprennent notamment l'autorisation d'accès, l'amorçage du dispositif, la posologie du produit contenu dans le réservoir, la sécurité contre le surdosage, la durée du traitement, la mise en veille et la sortie du mode veille, la position de fonctionnement, la sécurité enfant, et l'alerte du médecin traitant.

D'autres caractéristiques et avantages de la présente invention apparaîtront plus clairement au cours de la description détaillée suivante d'un mode de réalisation avantageux de celle-ci, faite en référence aux dessins joints, donnés à titre d'exemples non limitatifs, sur lesquels :
- la figure 1 est une vue schématique en perspective partiellement découpée d'un dispositif de pulvérisation selon la présente invention,
- la figure 2 est une vue similaire à la figure 1, montrant plus précisément les moyens de solidarisation selon la présente invention, et
- la figure 3 est un schéma montrant une utilisation avantageuse de la présente invention.

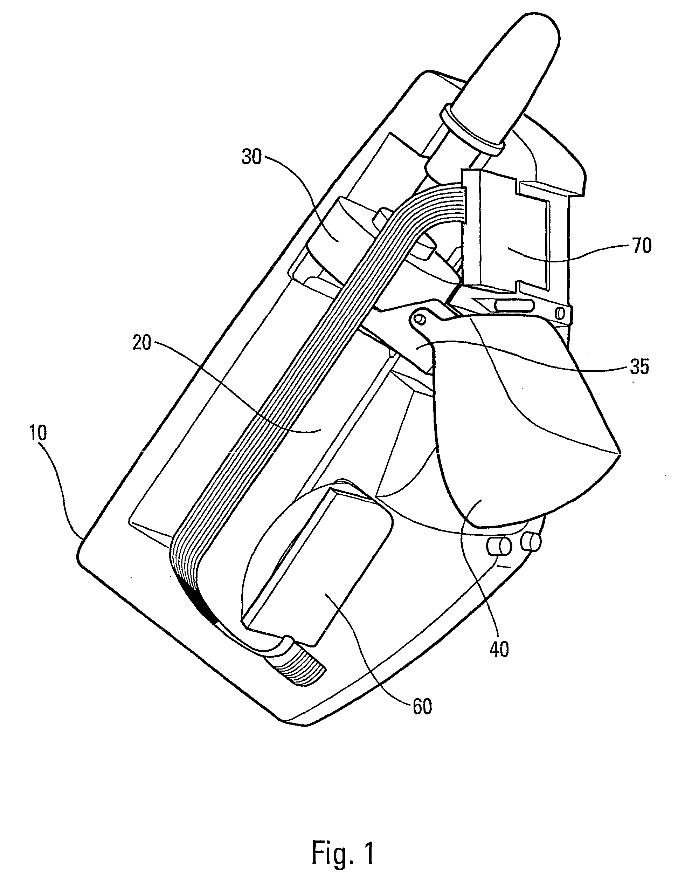

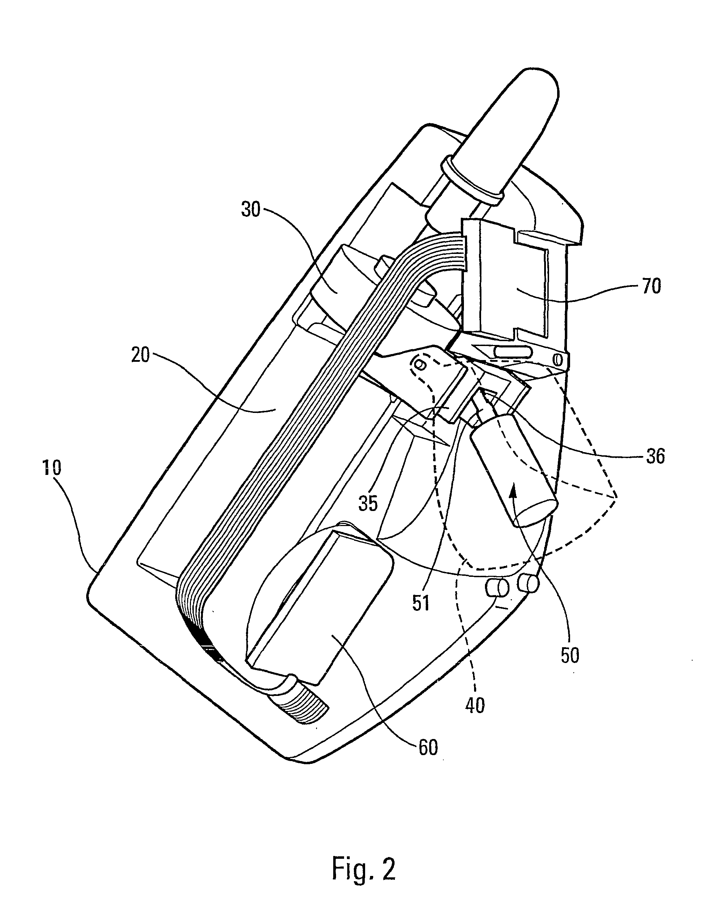

En référence aux figures, le dispositif de pulvérisation nasale ou orale de produit fluide ou pulvérulent comporte un corps 10 qui contient au moins un réservoir de produit 20 contenant une ou plusieurs doses de produit fluide ou pulvérulent à distribuer. Dans l'exemple représenté, le dispositif comporte un réservoir 20 contenant plusieurs doses. Des moyens de distribution 30, actionnés manuellement par l'utilisateur, tels qu'une pompe ou une valve, sont prévus pour distribuer sélectivement et de manière précise et reproductible des doses de produit contenues dans le réservoir 20. Dans l'exemple représenté sur les figures 1 et 2, les moyens de distribution sont réalisés sous la forme d'une pompe, mais d'autres moyens de distribution sont également envisageables, tels qu'une valve doseuse, ou des moyens de pulvérisation électronique ou électrotechnique, notamment piezzo électrique. Un élément d'actionnement manuel 40, de préférence réalisé sous la forme d'un bouton d'actionnement monté pivotant ou coulissant sur un côté latéral du corps 10 et sur lequel vient appuyer l'utilisateur, est utilisé pour actionner les moyens de distribution 30.

Selon l'invention, le dispositif comporte des moyens de solidarisation 50 qui sont mobiles entre une position embrayée dans laquelle l'élément d'actionnement manuel 40 coopère avec les moyens de distribution 30, et une position débrayée dans laquelle l'élément d'actionnement manuel 40 ne coopère pas avec lesdits moyens de distribution 30. Plus précisément, dans la position embrayée, un actionnement par l'utilisateur du bouton d'actionnement manuel 40 entraîne la distribution d'une dose de produit alors que dans la position débrayée, un actionnement du bouton d'actionnement 40 n'a aucun effet sur les moyens de distribution 30, et n'entraîne donc aucune distribution de produit. L'actionnement manuel du dispositif est donc bloqué par lesdits moyens de solidarisation 50 en position débrayée. Selon l'invention, ce système de solidarisation 50 est commandé par une unité de commande électronique paramétrable 60. Cette unité de commande 60, qui comporte de préférence un microprocesseur et des moyens d'alimentation en énergie adaptés, agit donc pour déplacer le système de solidarisation 50 entre sa position embrayée et sa position débrayée.

En référence plus particulièrement à la figure 2, le système de solidarisation 50 comporte avantageusement une tige 51, qui, dans la position embrayée, coopère avec un orifice de réception 36 prévu dans un organe 35 solidaire des moyens de distribution 30. Cet organe 35 solidaire des moyens de distribution 30 peut être relié au piston de la pompe, respectivement à la soupape de la valve, ou plus généralement à toute partie de produit des moyens de distribution 30 générant la distribution. Le système de solidarisation 50, et plus particulièrement la tige 51, est avantageusement disposé à l'intérieur du bouton d'actionnement 40, comme représenté sur la figure 2 sur laquelle le bouton 40 est représenté en pointillés. De cette manière, le dispositif peut utiliser un réservoir, une pompe et un système d'actionnement classique, la présence du système de solidarisation n'impliquant aucune modification particulière de ces éléments, seul l'organe 35 solidaire des moyens de distribution 30 en particulier de la pompe, étant légèrement modifié pour s'adapter à la réception de la tige en position embrayée.

Avantageusement, le dispositif comporte des moyens de signalisation 70, et en particulier un afficheur LCD électronique et/ou des avertisseurs sonores, pour informer l'utilisateur de l'état du dispositif et des différentes fonctions de celui-ci.

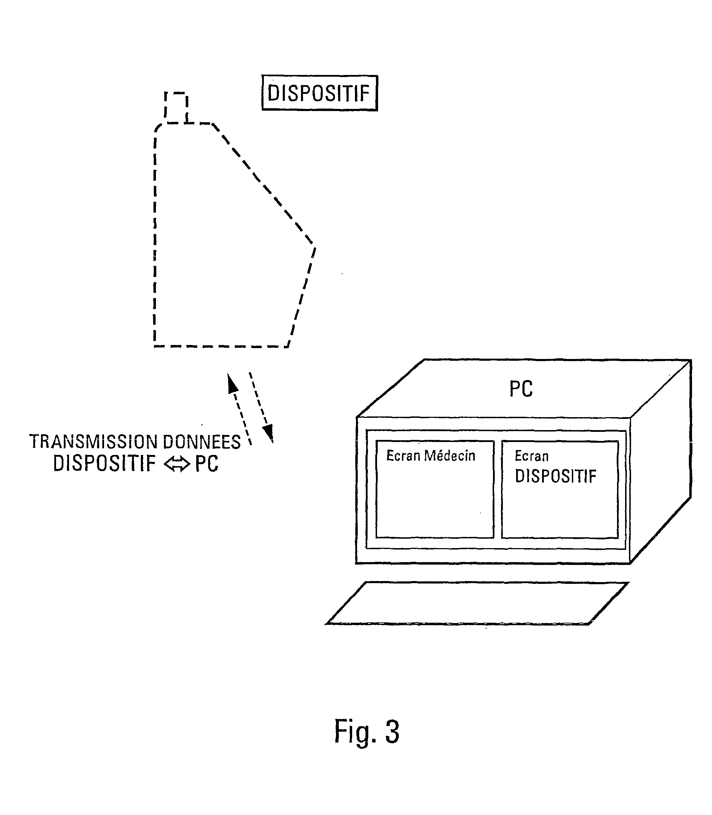

Comme mentionné précédemment, l'unité de commande 60 est paramétrable, ceci pouvant de préférence être réalisé à distance, de sorte que le dispositif comporte des moyens de transmission et de réception de données. La figure 3 représente schématiquement une telle transmission de données entre un dispositif de distribution et un ordinateur PC, qui peut être l'ordinateur du médecin traitant. Les paramètres de l'unité de commande électronique 60 sont modifiables, en particulier par le médecin, et peuvent être prédéterminés pour réaliser un certain nombre de fonctions. Ces fonctions peuvent notamment comprendre :
1. Instruction de l'utilisation de l'appareil : celles-ci peuvent être inscrites dans l'unité de commande électronique du dispositif au moyen de l'ordinateur PC, et peuvent comprendre des indications des étapes de mise en service, la durée du traitement, la posologie, etc.
2. Témoin de première utilisation : la mise en marche du dispositif peut être réalisée par pelage d'une languette de sécurité qui est disposée par exemple entre la pile et l'unité de commande avant la première utilisation. Avant retrait de la languette, l'actionneur latéral 40 est en position débrayée de sorte qu'un actionnement de celui-ci n'entraîne aucune distribution de produit. Ceci assure également une sécurité de transport.
3. Amorçage de l'appareil: le ou les premiers actionnements (en particulier les trois premiers actionnements) du dispositif peuvent correspondre à l'amorçage. Ceci peut s'afficher sur l'écran LCD, et des diodes de couleurs peuvent être prévues sur le dispositif pour signaler à l'utilisateur que le dispositif est en cycle d'amorçage.
4. Indiquer au patient quand prendre sa dose : un signal, notamment sonore, peut être émis par le dispositif ensemble avec un message qui s'affiche sur l'écran d'affichage LCD du dispositif.
5. Afficher/stocker le nombre de doses restantes : le stockage se fait dans une mémoire de l'unité de commande, et l'affichage se fait sur l'écran LCD du dispositif. Cet affichage peut comprend le nombre de doses restantes et le temps restant avant l'autorisation d'une prise de doses suivante. L'écran dispositif de l'ordinateur PC peut également afficher la durée du traitement.
6. Mise en veille automatique : celle-ci peut être effective après un certain nombre de minutes d'inutilisation, qui peut être ajusté au moyen de l'ordinateur PC. Cette mise en veille peut en particulier concerner les diodes, l'affichage LCD et le microprocesseur.
7. Sortie de mise en veille automatique : la sortie du mode veille peut se faire en deux temps, à savoir d'abord une réactivation de l'afficheur, et deuxièmement la demande d'un code pour faire fonctionner l'appareil.
8. Possibilité pour le patient de prendre une dose après une période autorisée en cas d'oubli : si, pour une posologie donnée, par exemple une prise toutes les quatre heures (T0, T0 + 4, T0 + 8, ...), la dose T0 + 4 est oubliée, alors il y a la possibilité pour le patient de prendre cette dose avant le temps T0 + 8 (par exemple T0 + 7). Dans ce cas, il y a recalage de l'espacement (quatre heures entre les prises) des doses suivantes sur la nouvelle heure T0 + 7, soit T0 + 11, T0 + 15, etc... Cet espacement peut notamment être ajusté au moyen de l'ordinateur PC.
9. Verrouillage entre deux doses : le système est en position débrayée entre deux doses pour empêcher un surdosage. L'afficheur sur l'écran dispositif de l'ordinateur indique le temps restant jusqu'à la prochaine dose, ce temps s'affichant également sur l'écran LCD du dispositif. Une diode rouge peut également s'allumer pendant cette période de verrouillage. Le dispositif peut en outre prévoir la saisie d'un code de déverrouillage si nécessaire.
10. Vérification de la position correcte du dispositif pendant la distribution de la dose: afin de garantir une position de fonctionnement d'environ 30° par rapport à l'axe vertical, le dispositif peut interdire l'actionnement de la pompe si la position n'est pas correcte, par exemple au moyen d'un capteur inclinomètre. Dans ce cas, un message peut s'afficher sur l'écran de l'ordinateur et/ou du dispositif pour prévenir l'utilisateur que la position est incorrecte.
11. Transmission des données vers le médecin pour connaître l'historique de prise de doses : les données stockées dans le dispositif peuvent être transmises vers l'ordinateur PC. Le médecin peut alors avoir accès à toutes les informations concernant les prises de doses par l'utilisateur du dispositif.
12. Modification de la posologie par le médecin : le temps entre deux doses peut être modifié par le médecin au moyen de l'ordinateur PC.
13. Alerte du médecin quand il reste moins de X doses dans l'appareil : ceci peut être réalisé au moyen d'un affichage sur l'écran médecin de l'ordinateur PC et le nombre de doses X à partir duquel cet affichage se fait peut être réglé par l'ordinateur PC. D'autre part, s'il reste très peu de doses, par exemple cinq, un message peut s'afficher sur l'écran dispositif de l'ordinateur et/ou l'écran LCD du dispositif, et le dispositif peut émettre un son strident au moyen d'un avertisseur sonore.
14. Sécurité enfant : l'accès au dispositif peut être régulé par un code (similaire à la sortie du mode veille).
15. Inviolabilité du système après la dernière dose (fin de traitement) : en fin d'utilisation, le dispositif peut être verrouillé et un affichage peut s'afficher sur l'affichage du dispositif et de l'ordinateur PC.
16. Possibilité de recharger le prototype en fin d'utilisation : le réservoir et la pile peuvent être rechargés et les différents paramètres peuvent être remis à zéro au moyen de l'ordinateur PC.

Bien entendu, les fonctions décrites ci-dessus ne sont données qu'à titre d'exemples pour un dispositif particulier, mais diverses autres fonctions sont envisageables, l'utilisation de l'électronique permettant aisément d'ajouter de nouvelles fonctions au dispositif.

La caractéristique essentielle de la présente invention réside dans la simplicité des moyens de solidarisation, réalisée au moyen d'une simple tige disposée à l'intérieur du bouton d'actionnement manuel et venant s'embrayer ou se débrayer avec un organe qui actionne la pompe de distribution. Cette caractéristique et cette mise en oeuvre permettent de réaliser un dispositif complet, transportable, au dosage précis et reproductible, et électroniquement paramétrable sans avoir à modifier les composants du dispositif de pulvérisation nasale ou orale de produit fluide ou pulvérulent en lui-même, c'est à dire le réservoir, la pompe ou l'élément d'actionnement manuel.

## Revendications

1. Dispositif de pulvérisation nasale ou orale de produit fluide ou pulvérulent comportant un corps (10), au moins un réservoir de produit (20) contenant une ou plusieurs doses de produit à distribuer, des moyens de distribution (30) actionnés manuellement, tels qu'une pompe ou une valve, et un élément d'actionnement manuel (40) adapté à actionner lesdits moyens de distribution (30) pour distribuer des doses de produit, **caractérisé en ce que** ledit dispositif comporte un système de solidarisation (50) qui est mobile entre une position embrayée, dans lequel ledit élément d'actionnement manuel (40) coopère avec lesdits moyens de distribution (30), et une position débrayée, dans laquelle ledit élément d'actionnement manuel (40) ne coopère pas avec lesdits moyens de distribution (30), ledit système de solidarisation (50) étant commandé par une unité de commande électronique paramétrable (60).

2. Dispositif selon la revendication 1, dans lequel ledit système de solidarisation (50) comporte un élément mécanique (51), tel qu'une tige, mobile entre une position embrayée dans laquelle il relie mécaniquement ledit élément d'actionnement (40) avec un organe (35) solidaire des moyens de distribution (30) de telle sorte qu'un actionnement dudit élément d'actionnement (40) entraîne la distribution d'une dose de produit, et une position débrayée dans laquelle il n'y a pas de liaison entre l'élément d'actionnement (40) et les moyens de distribution (30), de telle sorte qu'un actionnement dudit élément d'actionnement (40) n'entraîne pas de distribution de produit.

3. Dispositif selon la revendication 2, dans lequel ledit élément d'actionnement (40) est un bouton monté pivotant ou coulissant sur un côté latéral dudit corps (10), ladite tige (51) étant disposée dans ledit bouton d'actionnement (40), une extrémité de ladite tige (51) coopérant, dans la position embrayée, avec un orifice de réception (36) dudit organe (35) solidaire des moyens de distribution (30).

4. Dispositif selon l'une quelconque des revendications précédentes, dans lequel lesdits moyens de distribution (30) sont réalisés sous la forme d'une pompe ou d'une valve, ledit organe (35) solidaire des moyens de distribution (30) étant relié au piston de la pompe, respectivement à la soupape de la valve.

5. Dispositif selon l'une quelconque des revendications précédentes, dans lequel ladite unité de commande (60) comporte un microprocesseur et des moyens d'alimentation en énergie.

6. Dispositif selon l'une quelconque des revendications précédentes, dans lequel ledit dispositif comporte des moyens de signalisation (70), tels qu'un afficheur LCD ou des avertisseurs sonores, pour informer l'utilisateur de l'état du dispositif.

7. Dispositif selon l'une quelconque des revendications précédentes, dans lequel ladite unité de commande (60) est paramétrable à distance, notamment au moyen d'un ordinateur (PC), et comporte des moyens de transmission et de réception de données.

8. Dispositif selon l'une quelconque des revendications précédentes, dans lequel ladite unité de commande (60) est paramétrable pour permettre respectivement empêcher, l'actionnement du dispositif selon plusieurs fonctions prédéterminables.

9. Dispositif selon la revendication 8, dans lequel lesdites fonctions prédéterminables comprennent notamment l'autorisation d'accès, l'amorçage du dispositif, la posologie du produit contenu dans le réservoir, la sécurité contre le surdosage, la durée du traitement, la mise en veille et la sortie du mode veille, la position de fonctionnement, la sécurité enfant et l'alerte du médecin traitant.

## Patentansprüche

1. Vorrichtung zur nasalen bzw. oralen Zerstäubung eines Fluid- bzw. pulverförmigen Erzeugnisses, aufweisend einen Körper (10), zumindest einen Vorratsbehälter (20) für das Erzeugnis, welches mehrere Dosen des zu verteilenden Erzeugnisses enthält, Verteilungsmittel (30), die manuell betätigt werden, wie etwa eine Pumpe oder ein Ventil, und ein manuelles Betätigungselement (40), das dazu ausgelegt ist, die Verteilungsmittel (30) zur Verteilung der Erzeugnisdosen zu betätigen, **dadurch gekennzeichnet, dass** die Vorrichtung ein Festsetzsystem (50) umfasst, welches zwischen einer eingerückten Stellung, in welcher das manuelle Betätigungselement (40) mit den Verteilungsmitteln (30) zusammenwirkt, und einer ausgerückten Stellung beweglich ist, in welcher das manuelle Betätigungselement (40) nicht mit den Verteilungsmitteln (30) zusammenwirkt, wobei das Festsetzsystem (50) durch eine parametrierbare elektronische Steuereinheit (60) gesteuert ist.

2. Vorrichtung nach Anspruch 1, wobei das Festsetzsystem (50) ein mechanisches Element (51), wie etwa eine Stange umfasst, das zwischen einer eingerückten Stellung, in welcher es das Betätigungselement (40) mechanisch mit einem Organ (35) verbindet, das mit den Verteilungsmitteln (30) derart fest verbunden ist, dass eine Betätigung des Betätigungselements (40) die Verteilung der Erzeugnisdose mit sich bringt, und einer ausgerückten Stellung beweglich ist, in welcher es keine Verbindung zwischen dem Betätigungselement (40) und der Verteilungseinrichtung (30) herstellt, so dass eine Betätigung des Betätigungselements (40) keine Verteilung des Erzeugnisses zur Folge hat.

3. Vorrichtung nach Anspruch 2, wobei das Betätigungselement (40) eine Taste ist, die auf einer Lateralseite des Körpers (50) schwenkbar oder gleitend angebracht ist, wobei die Stange (51) in der Betätigungstaste (40) angeordnet ist, wobei ein Ende der Stange (51) in der eingerückten Stellung mit einer Aufnahmeöffnung (36) des Organs (35) zusammen wirkt, das mit den Verteilungsmitteln (30) fest verbunden ist.

4. Vorrichtung nach einem der vorangehenden Ansprüche, wobei die Verteilungsmittel (30) in Form einer Pumpe oder eines Ventils verwirklicht sind, wobei das Organ (35), das mit den Verteilungsmitteln (30) fest verbunden ist, mit dem Kolben der Pumpe bzw. dem Ventilorgan des Ventils verbunden ist.

5. Vorrichtung nach einem der vorangehenden Ansprüche, wobei die Steuereinheit (60) einen Mikroprozessor und Stromversorgungsmittel umfasst.

6. Vorrichtung nach einem der vorangehenden Ansprüche, wobei die Vorrichtung Signalgabemittel (70), wie etwa eine LCD-Anzeige oder Schallwarngabeeinrichtungen umfasst, um den Nutzer über den Zustand der Vorrichtung zu informieren.

7. Vorrichtung nach einem der vorangehenden Ansprüche, wobei die Steuereinheit (60) ferngesteuert parametrisierbar ist, insbesondere mittels eines Rechners (PC), und dass sie Mittel zur Übertragung und zum Empfangen von Daten umfasst.

8. Vorrichtung nach einem der vorangehenden Ansprüche, wobei die Steuereinheit (60) parametrisierbar ist, um die Betätigung der Vorrichtung in Übereinstimmung mit mehreren vorbestimmten Funktionen zu unterbinden.

9. Vorrichtung nach Anspruch 8, wobei die vorbestimmten Funktionen insbesondere die Zugangsautorisierung, die Initialisierung der Vorrichtung, die Posologie des Erzeugnisses, das in dem Vorratsbehälter enthalten ist, die Sicherheit gegenüber einer Überdosierung, die Behandlungsdauer, das Aktivieren einer Überwachungsbetriebsart und das Verlassen der Überwachungsbetriebsart, die Funktionsstellung, die Kindersicherung und die Alarmierung eines behandelnden Arztes umfassen.

## Claims

1. A fluid or powder nasal or oral spray device comprising a body (10), at least one fluid or powder reservoir (20) containing one or more doses of fluid or powder to be dispensed, manually actuated dispenser means (30), such as a pump or a valve, and a manual actuator element (40) adapted for actuating said dispenser means (30) to dispense doses of fluid or powder, said device being **characterized in that** it further comprises a securing system (50) which is mounted to move between a clutched position, in which said manual actuator element (40) co-operates with said dispenser means (30), and a declutched position, in which said manual actuator element (40) does not co-operate with said dispenser means (30), said securing system (50) being controlled by a parameterizable electronic control unit (60).

2. A device according to claim 1, in which said securing system (50) comprises a mechanical element (51), such as a rod, mounted to move between a clutched position in which it mechanically connects said actuator element (40) to a member (35) secured to the dispenser means (30) so that actuating said actuator element (40) causes a dose of fluid or powder to be dispensed, and a declutched position in which there is no coupling between the actuator element (40) and the dispenser means (30), so that actuating said actuator element (40) does not cause any fluid or powder to be dispensed.

3. A device according to claim 2, in which said actuator element (40) is a button mounted to pivot or slide on one side of said body (10), said rod (51) being disposed in said actuating button (40), one end of said rod (51) cooperating with an orifice (36), when said rod (51) is in the clutched position, which orifice serves to receive said member (35) secured to the dispenser means (30).

4. A device according to any preceding claim, in which said dispenser means (30) are implemented in the form of a pump or of a valve, said member (35) secured to the dispenser means (30) being connected to the piston of the pump, or to the valve member of the valve.

5. A device according to any preceding claim, in which said control unit (60) comprises a microprocessor and power supply means.

6. A device according to any preceding claim, in which said device further comprises indicator means (70) such as an LCD or sound alarms for informing the user of the state of the device.

7. A device according to any preceding claim, in which said control unit (60) can be parameterized remotely, in particular by means of a computer (PC), and further comprises means for transmitting and receiving data.

8. A device according to any preceding claim, in which said control unit (60) can be parameterized to enable and to inhibit actuation of the device through a plurality of predeterminable functions.

9. A device according to claim 8, in which said predeterminable functions comprise in particular access authorization, priming of the device, dosage of the fluid or powder contained in the reservoir, safety against overdosage, length of time of the treatment, shutting down to standby mode and coming out of standby mode, operating position, child safety and warning the patient's doctor.
